# EUROPEAN PATENT APPLICATION

(11) **EP 3 308 767 A1**
(43) Date of publication of application: **18.04.2018**
(21) Application number: 16807351.8
(22) Date of filing: 01.06.2016
(51) Int. Cl.: A61K 8/31, A61K 8/894, A61K 8/895, A61K 8/898, A61Q 1/04, A61Q 1/10, A61Q 5/00

(54) **COSMETIC**

(30) Priority: 10.06.2015 JP 2015117355
(71) Applicant: Shin-Etsu Chemical Co., Ltd., Tokyo 100-0004 (JP)
(72) Inventor: KONISHI Masayuki, Tokyo 100-0004 (JP); HAYAKAWA Chihiro, Tokyo 100-0004 (JP)
(74) Representative: V.O.
(86) International application number: PCT/JP2016/066233
(87) International publication number: WO 2016/199644

(57) **Abstract**

One of the purposes of the present invention is to provide a cosmetic composition having excellent adhesion. Specifically, the purpose of the present invention is to provide a cosmetic composition which provides the effect of making eyelashes longer and provides natural gloss on eyelashes without blending fibers. The present invention provides a cosmetic composition comprising a silicone-modified polysaccharide and an acryl-silicone graft copolymer. Further, the present invention provides a hair cosmetic composition, in particular a eyelash cosmetic composition.

## Description

### FIELD OF THE INVENTION

The present invention relates to a cosmetic composition comprising a silicone-modified polysaccharide as a film-forming agent and as a thread-forming thickener having good feeling in use. Specifically, the present invention relates to a hair cosmetic composition, such as an eyelash cosmetic such as a mascara. In the present specification, the cosmetic composition may be simply referred to as a cosmetic.

### BACKGROUND OF THE INVENTION

On account of various utilities, studies on compositions having a thread-forming property have been made in fields such as foods and resin, besides cosmetics and quasi drugs. A thread-forming property is such that a liquid having a high viscosity drops, or a stick is put into the liquid and pulled out from the liquid quickly and, then, the liquid results in the form of thread or string. A proper thread-forming property and viscosity are required in order to apply the composition to various applications.

A thread-forming composition is used in hair cosmetics, in particular eyelash cosmetics. An eyelash cosmetic is used in order to provide an effect of making eyes conspicuous by making eyelashes longer and beautiful. To this end, a long lash effect and a volume-boosting effect are attained by incorporating a thread-forming composition in an eyelash composition. For instance, Patent Literature 1 describes a thread-forming composition comprising both a lysine derivative-modified silicone and a silicone-modified polysaccharide, and the composition provides a long lash effect on eyelashes. Patent Literature 2 describes a technology of blending fibers and an adhesive film-forming agent in an eyelash cosmetic and applying the fibers to eyelashes to, thereby, increase the volume of eyelashes. Patent Literature 3 describes an oily gelling agent comprising a silicone-modified polysaccharide and a polyether-modified silicone, and a cosmetic comprising the oily gelling agent.

### PRIOR LITERATURE

Patent Literature 1: Japanese Patent Application Laid-Open No. 2007-314655
Patent Literature 2: Japanese National Phase Publication No. 2004-517092
Patent Literature 3: Japanese Patent Application Laid-Open No. 2008-105994

### SUMMARY OF THE INVENTION

### PROBLEMS TO BE SOLVED BY THE INVENTION

Eyelash cosmetic comprising fibers, as described in Patent Literature 2, has a high long lash effect, but provides an unnatural finish when the fiber is excessively applied to eyelashes. An oily gelling agent comprising a silicone-modified polysaccharide and a polyether-modified silicone, as described in Patent Literature 3, does not have sufficient viscosity nor gloss and, therefore, is insufficient as eyelash cosmetics.

One of the purposes of the present invention is to provide a cosmetic composition having an excellent adhesion. Specifically, the purpose of the present invention is to provide a cosmetic composition which provides an effect of making eyelashes appear longer without fibers and provides a natural glossy appearance on eyelashes.

### MEANS TO SOLVE THE PROBLEMS

The present inventors have conducted research and found that a cosmetic composition comprising a silicone-modified polysaccharide and an acryl-silicone graft copolymer provides a cosmetic composition having a good thread-forming property and excellent adhesion. Further, present inventors have found that an eyelash cosmetic comprising the cosmetic composition has excellent volume-boosting effect and long lash effect and provides an excellent gloss.

Thus, the present invention provides a cosmetic composition comprising a silicone-modified polysaccharide and an acryl-silicone graft copolymer. Further, the present invention provides a hair cosmetic composition, in particular an eyelash cosmetic composition.

### EFFECTS OF THE INVENTION

The present cosmetic composition has very good adhesion. Cosmetics obtained from the composition have an excellent volume-boosting effect on eyelashes and impart a long lash effect and gloss on eyelashes without blending fibers. Therefore, the present cosmetic composition is particularly suitable as hair cosmetics such as eyelash cosmetics.

### DETAILED DESCRIPTION OF THE INVENTION

The present invention will be described below in detail.

The present cosmetic composition is characterized in that the cosmetic composition comprises a silicone-modified polysaccharide and an acryl-silicone graft copolymer in combination. On account of the combination, the cosmetic composition has a good thread-forming property and provides a cosmetic having very excellent adhesion. In particular, when the cosmetic is applied to eyelashes, a voluminous feeling is attained. If either one of the silicone-modified polysaccharide and the acryl-silicone graft copolymer is lacking, the cosmetic composition obtained has a poor thread-forming property and poor adhesion. The ratio of the amount (parts by mass) of the silicone-modified polysaccharide to the amount (parts by mass) of the acryl-silicone graft copolymer is preferably 98:2 to 6:94 (or 49:1 to 1:15.7), more preferably 95:5 to 10:90 (or 19:1 to 1:9) in order to provide a cosmetic having better properties of forming a strong thread and an increased viscosity. When the present cosmetic composition is used for eyelashes, the ratio of the amount (parts by mass) of the silicone-modified polysaccharide to the amount (parts by mass) of the acryl-silicone graft copolymer is preferably 90:10 to 10:90 (or 9:1 to 1:9), more preferably 75:25 to 25:75 (or 3:1 to 1:3), in order to improve the volume-boosting effect, the long lash effect and the gloss of eyelashes.
Each component will be described below in detail.

### [Silicone-modified polysaccharide]

The silicone-modified polysaccharide has at least one silicone residue bonded to a hydroxyl group on a constituent sugar unit of the polysaccharide compound. This compound is obtained by reacting a hydroxyl group of a polysaccharide with a compound having a terminal isocyanate group borne by a silicone compound. The polysaccharide compound is preferably pullulan. The silicone residue is preferably one represented by the following general formula (1): 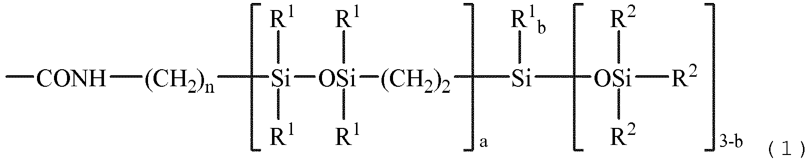 wherein R¹ is, independently of each other, an unsubstituted or substituted monovalent hydrocarbon group having 1 to 10 carbon atoms, R² is, independently of each other, an unsubstituted or substituted monovalent hydrocarbon group having 1 to 10 carbon atoms, or a siloxy group represented by -OSi(R³)₃, R³ is, independently of each other, an unsubstituted or substituted monovalent hydrocarbon group having 1 to 8 carbon atoms, n is an integer of from 1 to 10, a is 0 or 1 and b is 0, 1 or 2.

The average number of the silicone residue bonded to one constituent sugar unit of the polysaccharide (i.e., the degree of substitution) is 0.5 to 2.5, preferably 1 to 2. The constituent sugar unit is, for instance, a glucose unit in pullulan. If the average number is too small, the composition obtained does not have sufficient oil solubility. If the average number is too large, it is difficult to produce the compound itself.

The silicone-modified polysaccharide has an average molecular weight of 50,000 to 10,000,000, preferably an average molecular weight of 80,000 to 5,000,000. The average molecular weight is determined as the number average molecular weight or the weight average molecular weight, reduced to polystyrene, by gel permeation chromatography using a developing solvent such as toluene or tetrahydrofuran (THF).

Preferably, the silicone-modified polysaccharide has at least one silicone residue which is represented by the aforesaid formula (1) and which is bonded to a hydroxyl group on a glucose unit of pullulan. The average number of the silicone residue bonded to one glucose unit is 0.5 to 2.5, preferably 1 to 2.

In the above formula (1), examples of the unsubstituted or substituted monovalent hydrocarbon group, R¹, R² and R³, include alkyl groups such as a methyl group, an ethyl group, a propyl group, an isopropyl group, a butyl group, an isobutyl group, a tert-butyl group, a pentyl group, a hexyl group, a cyclohexyl group, a heptyl group, and an octyl group; alkenyl groups such as a vinyl group and an allyl group; aryl groups such as a phenyl group; aralkyl groups such as a benzyl group; and the foregoing hydrocarbon groups wherein a part or all of the hydrogen atoms bonded to a carbon atom is substituted with a substituent such as a halogen, atom such as a fluorine atom, a bromine atom and a chlorine atom, or a cyano group, e.g., a chloromethyl group, a chloropropyl group, a bromoethyl group, a trifluoropropyl group, and a cyanoethyl group. Among these, an unsubstituted or halogen-substituted monovalent hydrocarbon group is preferable, particularly an unsubstituted monovalent hydrocarbon group is preferable, and an alkyl group or an aryl group is more preferable. More particularly, a methyl group, an ethyl group and a phenyl group are preferable in view of the ease of synthesis and stability of the compound.

In the aforesaid formula (1), n is an integer of from 1 to 10, preferably an integer of from 1 to 6, a is 0 or 1, preferably 0, and b is 0, 1 or 2, preferably 0.

The silicone-modified polysaccharide is particularly represented by the following general formula (2): 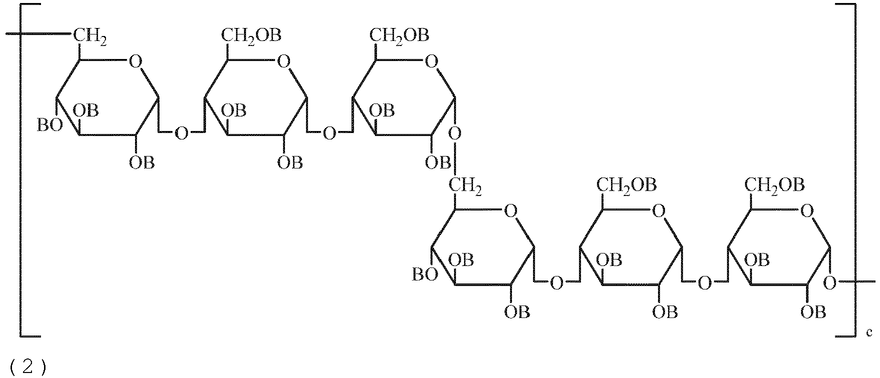 wherein, B is a hydrogen atom or a silicone residue represented by the formula (1), and c is a number of from 100 to 20,000.

Preferably, n is 3, a is 0, b is 0, and R² is a methyl group in the silicone residue represented by the aforesaid formula (1). That is, preferred is a compound represented by the aforesaid general formula (2) wherein B is a hydrogen atom or a group represented by -CONH(CH₂)₃Si[OSi(CH₃)₃]₃ and the average number of the silicone residue bonded to one glucose unit (i.e., the degree of substitution) is 0.5 to 2.5, preferably 1 to 2.

The present silicone-modified polysaccharide is obtained by reacting a hydroxyl group of a polysaccharide compound with a terminal isocyanate group borne by a silicone compound. For instance, the terminal isocyanate group-containing silicone compound represented by the following general formula (3) is reacted with a hydroxyl group of a polysaccharide compound, preferably pullulan. 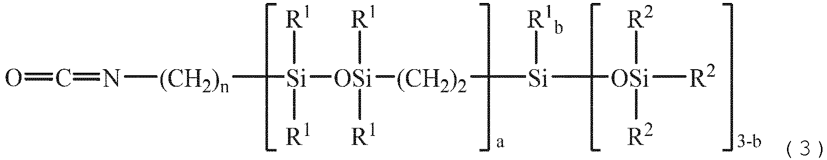 wherein R¹, R², n, a, and b are as defined above. The reaction may be conducted in any known manner. For instance, the method described in Japanese Patent Application Laid-Open No. Hei8-134103/1996 may be used. Specifically, pullulan and the compound represented by the formula (3) are mixed in a solvent, such as methyl acetate and N-methylpyrrolidone, and allowed to react at 20 to 150 degrees C for 1 to 10 hours. In the reaction, a catalyst, such as triethylamine, may be used to accelerate the formation of a urethane bond. The mixing ratio of pullulan and the compound represented by the formula (3) may depend on the amount to be bonded to the three hydroxyl groups per glucose unit of pullulan and may preferably be adjusted so that the average number of the silicone compound bonded to one glucose unit is 0.5 to 2.5.

The present silicone-modified polysaccharide may be a commercially available product, such as tri(trimethylsiloxy) silyl propyl carbamic acid pullulan (TSPL) which is a cosmetic label name. Examples of commercially available products include TSPL dissolved in decamethylcyclopentasiloxane which is marketed as TSPL-30-D5 from Shin-Etsu Chemical Co., Ltd. and TSPL dissolved in isododecane which is marketed as TSPL-30-ID from Shin-Etsu Chemical Co., Ltd.

The amount of the silicone-modified polysaccharide may be 0.1 to 25 mass %, preferably 0.3 to 15 mass %, more preferably 1.0 to 10 mass %, even more preferably 1.5 to 6 mass %, based on the total amount of cosmetic composition. If the amount is larger than the aforesaid upper limit, the feeling in use of the cosmetic is heavy. If the amount is less than the aforesaid lower limit, the cosmetic does not have a sufficient thread-forming property, a sufficiently increased viscosity, nor adhesion.

### [Acryl-silicone graft copolymer]

The acryl-silicone graft copolymer consists of an acrylic polymer and a polysiloxane. In particular, the acryl-silicone graft copolymer is obtained by grafting a dimethyl polysiloxane chain on an acrylic polymer chain. The graft copolymer whose trunk polymer is an acrylic polymer and the branch polymer is an organopolysiloxane is preferable in view of adhesion, flexibility of a film and gloss. Any known acryl-silicone graft copolymer may be used. The acryl-silicone graft copolymer may be used singly or in combination of two or more.

The acryl-silicone graft copolymer may be obtained, for example, by the reaction of an acrylic monomer with an organopolysiloxane having a radical-polymerizable group. The organopolysiloxane having a radical-polymerizable group, referred to also as a silicone macromonomer, is known and has a radical-polymerizable group at only one terminal. The organopolysiloxane may be dialkylorganopolysiloxane, particularly dimethylpolysiloxane. The degree of polymerization of the siloxane may be 3 to 500, preferably 9 to 200. Examples of the radical-polymerizable group include a (meth)acryloxy methyl group, a (meth) acryloxy propyl group, a (meth)acryloxy decyl group, a styryl group, and an α-methylstyryl group. A (meth)acryloxy propyl group is preferred.

The acrylic polymer chain is introduced by an acrylic monomer. Examples of the acrylic monomer include methyl(meth)acrylate, ethyl(meth)acrylate, isopropyl(meth)acrylate, n-butyl(meth)acrylate, t-butyl(meth)acrylate, cyclohexyl(meth)acrylate, 2-ethylhexyl(meth)acrylate, tridecyl(meth)acrylate, stearyl(meth)acrylate, isostearyl(meth)acrylate and behenyl(meth)acrylate. They may be used singly or in combination of two or more. Among these, methyl(meth)acrylate is preferable. Further, the carboxyl group in an acrylic acid, and the like, is preferably esterified so that the acrylic odor of the copolymer is reduced.

The copolymer may be further copolymerized with a radical-polymerizable silane compound. Examples of the silane compound include gamma-methacryloxypropyl trimethoxysilane, gamma-methacryloxypropyl methyl dimethoxy silane, gamma-methacryloxypropyl dimethyl methoxysilane, gamma-methacryloxypropyl triethoxysilane, gamma-methacryloxypropyl methyl diethoxy silane, gamma-methacryloxypropyl tributoxy silane, gamma-methacryloxypropyl triisopropenoxy silane, gamma-acryloxypropyl trimethoxy silane, gamma-acryloxymethyl trimethoxy silane, gamma-acryloxypropyl triethoxysilane, gamma-acryloxypropylmethyl diethoxy silane, styryltrimethoxysilane, styryltriethoxysilane, and alpha-methyl styryltrimethoxysilane. These may be used singly or in combination of two or more. Gamma-methacryloxypropyl triethoxysilane is preferred.

The amount of the component constituting the copolymer is not particularly limited. For instance, the amount of the organopolysiloxane having a radical-polymerizable group is 1 to 97 wt%, preferably 5 to 90 wt%, an amount of the acrylic monomer is 0.5 to 95 wt%, preferably 2 to 60 wt%, and the amount of the radical polymerizable silane compound is 0.5 to 10 wt%, preferably 2 to 7 wt%. If the amount of the organopolysiloxane is too small, the sufficient effects as a hair treatment agent are not obtained. If the amount of the organopolysiloxane is too large, adsorption to hair or reactivity is poor. If the amount of the acrylic monomer is too large, sufficient effects such as water resistance are not obtained.

In addition to the aforesaid components, the other radical polymerizable compound may be copolymerized in an amount such that the effects of the present invention are not obstructed. Examples of the other radical polymerizable compound include carboxylic acids such as (meth)acrylic acid, fumaric acid and maleic acid; hydroxyalkyl esters such as hydroxyethyl(meth)acrylate and hydroxypropyl(meth)acrylate; acid amides such as (meth)acrylamide; fluorine-substituted alkyl esters such as perfluorooctylethyl(meth)acrylate and perfluorobutyl ethyl(meth)acrylate; styrene, acrylonitrile, vinyl acetate, vinyl pyrrolidone, polyoxyethylene mono(meth)acrylate, polyoxypropylene mono(meth)acrylate, polyoxy(ethylene-propylene)mono(meth)acrylate, polycaprolactone mono(meth)acrylate, tris(trimethylsiloxy)silyl propyl methacrylate, and tris(trimethylsiloxy)silylstyrene.

The copolymerization method is not particularly limited. Any known method such as solution polymerization, emulsion polymerization, suspension polymerization and bulk polymerization may be applied. The solution polymerization is preferred in view of uniformity of the polymer obtained and easy control of the molecular weight of the polymer. A solvent which uniformly dissolves the aforesaid components and the polymer is preferably used in the copolymerization. Examples of the solvent include toluene, xylene, ethanol, isopropyl alcohol, n-butanol, acetone, methylethylketone, methylisobutylketone, ethyl acetate, n-butyl acetate, isobutyl acetate, diethyl ether, and tetrahydrofuran. The polymerization initiator may be any ordinary radical initiator, e.g., organic peroxides such as benzoyl peroxide and dicumyl peroxide, and azo compounds such as azobisisobutyronitrile.

The molecular weight of the acryl-silicone copolymer is not particularly limited. The weight average molecular weight is preferably 3,000 to 200,000, more preferably 5,000 to 100,000, as determined by GPC, and reduced to polystyrene. If the amount is less than the aforesaid lower limit, the effects in a treatment on hair is insufficient. If the amount is larger than the aforesaid upper limit, feeling in use of the cosmetic is poor.

The acryl-silicone graft copolymer is preferably a commercially available product. For instance, an (alkyl acrylate/dimethicone)copolymer which is a cosmetic label name may be used. Examples of the commercially available product include KP-543, KP-545, KP-549, KP-550 and KP-545L, as dissolved in a solvent, ex Shin-Etsu Chemical Co., Ltd.

The amount of the acryl-silicone graft copolymer may be 0.1 to 31 mass %, preferably 0.3 to 16 mass %, more preferably 1.0 to 10 mass %, even more preferably 1.5 to 6 mass %, based on the total mass of the cosmetic composition. If the amount is larger than the aforesaid upper limit, the feeling in use of a cosmetic is heavy. If the amount is less than the aforesaid lower limit, a sufficient thread-forming property, an increased viscosity and gloss are not provided in cosmetics.

### [Nonionic Surfactant]

The present cosmetic composition may further comprise a nonionic surfactant. Any nonionic surfactant may be used. Particularly, a silicone emulsifier is preferable. Any known silicone emulsifier may be used. Examples of the silicone emulsifier include polyoxyalkylene-modified silicone, polyglycerin-modified silicone and sugar alcohol-modified silicone. When a particularly high increased viscosity is desired, one or more selected from polyoxyalkylene-modified silicone and polyglycerin-modified silicone are preferred.

Examples of the polyoxyalkylene-modified silicone include PEG-11 methyl ether dimethicone, PEG/PPG-20/22 butyl ether dimethicone, PEG-3 dimethicone, PEG-9 methylether dimethicone, PEG-10 dimethicone, PEG-9 polydimethylsiloxyethyl dimethicone, lauryl PEG-9 polydimethylsiloxyethyl dimethicone, PEG/PPG-18/18 dimethicone, cetylPEG/PPG-10/1 dimethicone, (dimethicone/(PEG-10/15))crosspolymer, (PEG-15/lauryl dimethicone)crosspolymer, (PEG-10/lauryl dimethicone)crosspolymer and (PEG-15/lauryl dimethicone) crosspolymer, and (PEG-15/ lauryl polydimethylsiloxyethyl dimethicone) crosspolymer, which are cosmetic label names. These may be used as a mixture with any oil component.

Examples of commercially available products of these include KF-6011, KF-6011P, KF-6012, KF-6013, KF-6015, KF-6016, KF-6017, KF-6017P, KF-6028, KF - 6028P, KF-6038, KF-6048, KSG-210, KSG-240, KSG-310, KSG-320, KSG-330, KSG-340, KSG-320Z and KSG-350Z, all provided by Shin-Etsu Chemical Co., Ltd.

The polyglycerin-modified silicone may be, specifically, polyglyceryl-3 disiloxane dimethicone, polyglyceryl-3 polydimethylsiloxyethyl dimethicone, lauryl polyglyceryl-3 polydimethylsiloxyethyl dimethicone, bis-butyl dimethicone polyglyceryl-3, (dimethicone/polyglycerin-3)crosspolymer, (lauryl dimethicone/polyglycerin-3)crosspolymer, (polyglyceryl-3/lauryl polydimethylsiloxyethyl dimethicone) crosspolymer, which are cosmetic label names. These may be used as a mixture with any oil component.

Examples of commercially available products include KF-6100, KF-6104, KF-6105, KF-6106, KF-6109, KSG-710, KSG-810, KSG-820, KSG-830, KSG-840, KSG-820Z and KSG-850Z, all provided by Shin-Etsu Chemical Co., Ltd.

The amount of the nonionic surfactant may be 0.1 to 10 mass %, preferably 1.5 to 4 mass %, based on the total mass of the cosmetic composition. If the amount is less than the aforesaid lower limit, sufficient feeling of application is not obtained. If the amount is larger than the aforesaid upper limit, a uniform film is not obtained and, therefore, natural finish feeling may not be obtained.

### [Wax]

The present cosmetic composition may further contain a wax.
Any wax usually used in cosmetics may be used. For instance, synthetic hydrocarbon waxes such as ceresin, ozokerite, microcrystalline wax and polyethylene wax; wax of plant origin such as carnauba wax, rice wax, rice bran wax, jojoba wax (including extremely hydrogenated jojoba oil) and candelilla wax; wax of animal origin such as whale wax, beeswax and snow wax; and a silicone wax may be used. These may be used singly or in combination of two or more.

The amount of wax is 3 to 20 mass %, preferably 5 to 13 mass %, based on the total mass of the cosmetic composition. If the amount is less than the aforesaid lower limit, sufficient voluminous feeling may not be obtained. If the amount is larger than the aforesaid upper limit, a uniform film may not be obtained and, therefore, beautiful finishing may not be obtained.

### [Other Components]

The present cosmetic component preferably further contains a liquid oil, such as volatile oils and non-volatile oils. It is very important to choose volatility of the liquid oil in order to obtain a good usability of an eyelash cosmetic.

Any volatile oil usually used in cosmetics may be used. For instance, cyclopentasiloxane, cyclohexasiloxane, disiloxane, trisiloxane, dimethicone(1.5cs, 2cs), methyltrimethicone, isododecane, isohexadecane, undecane, and tridecane may be used. Examples of commercially available products of these include KF-96 series, KF-995 and TMF-1.5, ex Shin-Etsu Chemical Co., Ltd.

The present cosmetic composition may contain an organic-modified clay mineral, if needed. Any organic-modified clay mineral usually used in cosmetics may be used. Preferably, dimethyl distearyl ammonium hectorite, dimethyl distearyl ammonium bentonite, and dimethyl distearyl ammonium modified montmorillonite may be used, such as BENTONE38VCG (dimethyl distearyl ammonium hectorite) and BENTONE34 (dimethyl distearyl ammonium bentonite), ex ELEMENTIS SPECIALTIES. The organic-modified clay minerals may be used singly or in combination of two or more.

Further, the synthetic organic-modified bentonite may be one which is swelled in oil, such as BENTONE GEL ISDV, BENTONE GEL MIOV and BENTONE GEL VS-5V, ex ELEMENTIS SPECIALTIES.

The present cosmetic composition may further contain a carbonate ester, if needed. Any carbonate ester usually used in cosmetics may be used. For instance, cyclic carbonate ester and ring-opened carbonate ester can be used. More particularly, ethylene carbonate, dialkyl(C14,15)carbonate, di-ethylhexyl carbonate, dicaprylyl carbonate, butylene carbonate, and propylene carbonate, and propylene carbonate are preferable. These carbonates may be used singly or in combination of two or more.

The present cosmetic composition may further contain a film-forming agent, if needed. Any film-forming agent usually used in cosmetics may be used, including latexes such as polyvinyl alcohol, polyvinyl pyrrolidone, polyvinyl acetate, alkyl polyacrylate; cellulose derivatives such as dextrin, alkyl cellulose and nitrocellulose; silicone resins such as trimethylsiloxy silicate; silicone-modified polynorbornene, silicone resins such as fluorine-modified silicone resin, fluorine resin, aromatic hydrocarbon resin, polymer emulsion resins, terpene resins, polybutene, polyisoprene, alkyd resins, polyvinylpyrrolidone-modified polymers, rosin-modified resins, and polyurethane.

Among these, a silicone-based film-forming agent is preferred, such as trimethylsiloxysilicate, e.g., KF-7312J and X-21-5250, ex Shin-Etsu Chemical Co., Ltd., and silicone-modified polynorbornene, e.g., NBN-30-ID, ex Shin-Etsu Chemical Co., Ltd. These film-forming agents may be used singly or in combination of two or more.

The present cosmetic composition may contain a pigment, if needed. Any pigment usually used in cosmetics may be used, such as inorganic pigments such as talc, mica, kaolin, silica, calcium carbonate, zinc oxide, titanium dioxide, red iron oxide, yellow iron oxide, black iron oxide, ultramarine, iron blue, carbon black, lower titanium oxide, cobalt violet, chromium oxide, chromium hydroxide, cobalt titanate, bismuth oxychloride, and mica-based titanium pearl pigment; organic pigments having zirconium, barium or aluminum lake, e.g., red No.201, red No.202, red No.204, red No.205, red No.220, red No.226, red No.228, red No.405, orange No.203, yellow No.205, yellow No.4, yellow No.5, blue No.1, blue No.404 and green No.3; natural pigments such as chlorophyll or beta-carotene; and dyes may be used. They may be hydrophobized with a silicone. These pigments may be used singly or in combination of two or more.

When the present cosmetic composition is used as a hair cosmetic such as an eyelash cosmetic, fibers may be contained in the composition, if needed. Fibers impart a long lash effect on eyelashes, make eyelashes look longer and form a uniform cosmetic film. Any fibers usually used in cosmetics may be used, such as synthetic fibers such as nylon and polypropylene; artificial fibers such as rayon; natural fibers such as cellulose; and semi-synthetic fibers such as acetate rayon. The thickness of these fibers may be 3 to 12 deniers (hereinafter, simply referred to as "D") and the length may be 0.1 to 4 mm, in view of the effect of brightening eyes.

The fibers may be surface-treated, if needed. Examples of a surface-treating agent include fluorine compounds, silicone compounds, powder, oil, gelling agent, polymer emulsions and surfactants. These may be used singly or in combination of two or more. Among these, the fibers treated with a silicone or a fluorine are preferable because of the effects of making the eyelashes appear thicker and longer, providing the effect of brightening eyes and of sustainment of these effects.

The present cosmetic composition may contain a sugar fatty acid ester, if needed. The sugar fatty acid ester provides effects of increasing the viscosity, gelling cosmetics and improving adhesion and stability at a high temperature. Examples of commercially available products include Rheopearl KL2, Rheopearl TL2, Rheopearl TT2, Rheopearl MKL2 and Rheopearl ISK2, ex Chiba Flour Milling Co., Ltd., but is not limited to these. The sugar fatty acid ester may be used singly or in combination of two or more.

The present cosmetic composition may contain oil-absorbing powder, if needed. For instance, silicone composite powder such as KSP-100, KSP-300, KSP-411 and KSP-441, ex Shin-Etsu Chemical Co., Ltd.; silicone rubber powder such as KMP-598, ex Shin-Etsu Chemical Co., Ltd.; and porous powder such as nylon powder may be used. Specifically, silica microparticle having a high specific surface area are typically used in view of increasing the viscosity and gelling cosmetics, e.g., silica fine powder having a BET specific surface area of 50 m²/g or more, particularly 100 to 400 m²/g, such as fumed silica and precipitated silica. Examples of the microparticle silica include Aerosil R972 and Aerosil 200, ex Nippon Aerosil Co. The aforesaid oil-absorbing powder is blended to absorb the solvent in a film-forming agent to, thereby, increase the concentration of the film-forming agent and to control the thread-forming property, increase of the viscosity of the composition and the covering feel.

The present cosmetic composition may be used in various cosmetics products such as skin milks, creams, foundations, lipsticks, eye shadows, eyeliners, body makeup preparations, and hair cosmetics, but is not limited to these. Further, the present cosmetic composition may be used in non-aqueous oily cosmetics such as a gloss, a stick concealer and a pouring foundation. In particular, it is preferably used as a hair cosmetic which is used for human hair such as hair of the head, eyebrows and eyelashes. For instance, the hair cosmetic may be a hair setting agent, a hair cream and a mascara.

### EXAMPLES

The present invention will be explained in detail with reference to the following Examples and Comparative Examples, but is not limited to them.

In the following, the amount is in mass% unless otherwise specified.

The thread-forming property and the increase of viscosity in Tables 1 and 2 were evaluated as follows.

A bar is put into the cosmetic composition and then quickly pulled up. It is observed how the composition forms a thread. When strong thread was formed, the thread-forming property is rated as A. When very strong thread was formed, the thread-forming property is rated as AA. When weak thread was formed, the thread-forming property is rated as B. When the composition did not form any thread, the composition was rated as C.

The following components were mixed. It is observed how the viscosity of the mixture increased, compared to the viscosities of the raw materials before being mixed. When the viscosity increased well, increased viscosity property is rated as A. When the viscosity increased considerably, the property of increased viscosity is rated as AA. When the viscosity slightly increased, the property of increasing viscosity is rated as B. When the viscosity did not increase, the property of increased viscosity is rated as C.

The viscosity of the wax-blended composition increased remarkably by forming a crystal structure of the wax and, therefore, the evaluation of the property of increased viscosity was not conducted.

### [Examples 1 to 7 and Comparative Examples 1 to 4]

The oily cosmetic compositions were prepared in the compositions as shown in Table 1. The thread-forming property and the property of increasing viscosity of each composition were evaluated in accordance with the aforesaid criteria.

**[Table 1]**

| Component, Parts by mass | Example | | | | | | | Comparative Example | | | |
|---|---|---|---|---|---|---|---|---|---|---|---|
| | 1 | 2 | 3 | 4 | 5 | 6 | 7 | 1 | 2 | 3 | 4 |
| (A)Silicone-modified polysaccharide solution A (Note 1) | 10 | 10 | 19.8 | 19 | 18 | 2 | 1 | 20 | 20 | - | - |
| (B)Acryl-silicone graft copolymer solution A (Note 2) | 10 | 10 | 0.2 | 1 | 2 | 18 | 19 | - | - | 20 | 20 |
| Ceresin | - | 5 | - | - | - | - | - | - | 5 | - | 5 |
| Isododecane | Balance | - | Balance | Balance | Balance | Balance | Balance | Balance | - | Balance | - |
| Total amount, Parts by mass | 25 | 25 | 25 | 25 | 25 | 25 | 25 | 25 | 25 | 25 | 25 |
| Ratio of the amounts (A) : (B) | 1:1 | 1:1 | 99:1 | 19:1 | 9:1 | 1:9 | 1:19 | 1:0 | 1:0 | 0:1 | 0:1 |
| Thread-forming property | A | AA | A | A | A | A | A | C | B | C | C |
| Viscosity increase | A | - | B | A | A | A | B | C | - | C | - |

| | | | | | | | | | | | |
|---|---|---|---|---|---|---|---|---|---|---|---|
| Note 1:Silicone-modified polysaccharide solution A: TSPL-30-ID/a solid content 30 %, ex Shin-Etsu Chemical Co., Ltd. Note 2:Acryl-silicone graft copolymer solution A: KP-550/a solid content 40 %, ex Shin-Etsu Chemical Co., Ltd. | | | | | | | | | | | |

When the cosmetic composition is applied on eyelashes, it is important that the cosmetic composition has excellent adhesion in order to give a voluminous appearance to the eyelashes. A strong thread-forming property and a controlled viscosity are needed to control the adhesion. As seen in Comparative Examples 1 to 4 in Table 1, when either one of the silicone-modified polysaccharide and the acryl-silicone graft copolymer was lacking, the cosmetic composition obtained has poor thread-forming property and poor increasing viscosity property. In contrast, the cosmetic composition of the present invention has good thread-forming property. In particular, when the ratio of the amount of the silicone-modified polysaccharide to the amount of the acryl-silicone graft copolymer is 19:1 to 1:9, the composition has a good thread-forming property and its viscosity can be easily controlled. When the ratio of the amounts is 99:1 or 1:19, the composition has good thread-forming property, but the increasing viscosity property is slightly poor. Therefore, a more preferable ratio of the amount of the silicone-modified polysaccharide to the amount of the acryl-silicone graft copolymer is 19:1 to 1:9.

As seen in Comparative Example 2, the cosmetic composition has good thread-forming property on account of the combination of the wax and the silicone-modified polysaccharide, but its thread-forming property is inferior compared to the present cosmetic compositions.

### [Examples 8-10 and Comparative Examples 5-7]

The oily cosmetic compositions were prepared in the compositions as shown in Table 2. The thread-forming property and the property of increasing viscosity of each composition were evaluated in accordance with the aforesaid criteria.

**[Table 2]**

| Component, Parts by mass | Example | | | | Comparative Example | | |
|---|---|---|---|---|---|---|---|
| | 1 | 8 | 9 | 10 | 5 | 6 | 7 |
| (A)Silicone-modified polysaccharide solution A ^{(Note 1)} | 10 | 10 | 10 | 10 | 20 | 20 | 20 |
| (B)Acryl-silicone graft copolymer solution A ^{(Note 2)} | 10 | 10 | 10 | 10 | - | - | - |
| Linear polyether-modified silicone A ^{(Note 3)} | - | 2 | - | - | 2 | - | - |
| Linear polyether-modified silicone B ^{(Note 4)} | - | - | 2 | - | - | 2 | - |
| Linear polyether-modified silicone C ^{(Note 5)} | - | - | - | 2 | - | - | 2 |
| Isododecane | Balance | Balance | Balance | Balance | Balance | Balance | Balance |
| Total amount, Parts by mass | 25 | 25 | 25 | 25 | 25 | 25 | 25 |
| HLB of activator | - | 4.0 | 7.0 | 14.5 | 4.0 | 7.0 | 14.5 |
| Thread-forming property | A | AA | AA | AA | B | B | B |
| Viscosity increase | A | A | A | A | B | B | B |

| | | | | | | | |
|---|---|---|---|---|---|---|---|
| Note 3:Linear polyether-modified silicone A: KF-6017P, ex Shin-Etsu Chemical Co., Ltd. Note 4:Linear polyether-modified silicone B: KF-6012, ex Shin-Etsu Chemical Co., Ltd. Note 5:Linear polyether-modified silicone C: KF-6011P, ex Shin-Etsu Chemical Co., Ltd. | | | | | | | |

As seen in Comparative Examples 5-7 in Table 2, the cosmetic composition which did not comprise the acryl-silicone graft copolymer had poor thread-forming property and poor increasing viscosity. In contrast, the cosmetic composition of the present invention had good thread-forming property and increasing viscosity. In particular, as seen from a comparison between the composition of Example 1 and the compositions of Examples 8 to 10, when the nonionic surfactant is further added to the mixture of the silicone-modified polysaccharide and the acryl-silicone graft copolymer, thread-forming property of the cosmetic composition is further improved and a stronger thread is formed. This effect is achieved in a wide range of the HLB 4.0 to 14.5.

### [Examples 11-15 and Comparative Examples 8 and 9]

The eyelash cosmetics were prepared in the compositions as shown in Table 3. The voluminous appearance, the long lash effect and gloss of each cosmetic were evaluated according to the following criteria.

### (1) Voluminous appearance

Each of the eyelash cosmetics was applied 5 times on the eyelashes of 10 experienced panelists, and the volume of eyelashes was visually observed before and after application and the volume was evaluated. When the volume of eyelashes was considerably increased, the eyelash cosmetic was given 5 points. When the volume of eyelashes was slightly increased, the eyelash cosmetic was given 3 points. When the volume did not change, the eyelash cosmetic was given 1 point. The total of the scores given by the 10 experienced panelists was calculated and rated according to the following criteria. The results are as shown in Table 3.
A: Total point is 40 points or more
B: Total point is 21 to 39 points
C: Total point is 20 points or less

### (2) Long lash effect

Each of the eyelash cosmetics was applied 5 times on the eyelashes of 10 experienced panelists, and the length of the eyelashes was visually observed before and after application and the long lash effect was evaluated. When the eyelash became conspicuously longer, the eyelash cosmetic was given 5 points. When the eyelash became slightly longer, the eyelash cosmetic was given 3 points. When the length of the eyelashes did not change, the eyelash cosmetic was given 1 point. The total of the scores given by the 10 experienced panelists was calculated and rated according to the following criteria. The results are as shown in Table 3.
A: Total point is 40 points or more
B: Total point is 21 to 39 points
C: Total point is 20 points or less

### (3) Gloss

Each of the eyelash cosmetics was applied 5 times on the eyelashes of 10 experienced panelists, and the gloss of the eyelashes was visually observed before and after application and the gloss was evaluated. When the gloss of eyelash was considerably improved, the eyelash cosmetic was given 5 points. When the gloss of eyelash was slightly improved, the eyelash cosmetic was given 3 points. When the gloss of the eyelashes did not change, the eyelash cosmetic was given 1 point. The total of the scores given by the 10 experienced panelists was calculated and rated according to the following criteria. The results are as shown in Table 3.
A: Total point is 40 points or more
B: Total point is 21 to 39 points
C: Total point is 20 points or less

**[Table 3]**

| Component, Parts by mass | Example | | | | | Comparative Example | |
|---|---|---|---|---|---|---|---|
| | 11 | 12 | 13 | 14 | 15 | 8 | 9 |
| (A)Silicone-modified polysaccharide solution A ^{(Note 1)} | 10 | 18 | 15 | 5 | 2 | 20 | 0 |
| (B)Acryl-silicone graft copolymer solution A ^{(Note 2)} | 10 | 2 | 5 | 15 | 18 | 0 | 20 |
| Branched polyether-modified silicone ^{(Note 6)} | 1.5 | 1.5 | 1.5 | 1.5 | 1.5 | 1.5 | 1.5 |
| Organic-modified clay mineral ^{(Note 7)} | 5 | 5 | 5 | 5 | 5 | 5 | 5 |
| Carnauba Wax | 6 | 6 | 6 | 6 | 6 | 6 | 6 |
| Beeswax | 6 | 6 | 6 | 6 | 6 | 6 | 6 |
| Propylene carbonate | 2 | 2 | 2 | 2 | 2 | 2 | 2 |
| Isododecane | Balance | Balance | Balance | Balance | Balance | Balance | Balance |
| Silicone-treated ^{(Note 8)} black iron oxide | 5 | 5 | 5 | 5 | 5 | 5 | 5 |
| Silicone-treated ^{(Note 8)} talc | 5 | 5 | 5 | 5 | 5 | 5 | 5 |
| Preservative | Proper quantity | Proper quantity | Proper quantity | Proper quantity | Proper quantity | Proper quantity | Proper quantity |
| Total amount, Parts by mass | 100 | 100 | 100 | 100 | 100 | 100 | 100 |
| Ratio of the amounts (A) : (B) | 1:1 | 9:1 | 3:1 | 1:3 | 1:9 | 1:0 | 0:1 |
| Voluminous appearance | A | A | A | A | B | B | C |
| Long lash effect | A | A | A | A | A | A | C |
| Gloss | A | B | A | A | A | C | A |

| | | | | | | | |
|---|---|---|---|---|---|---|---|
| Note 6: Branched polyether-modified silicone: KF-6028P, ex Shin-Etsu Chemical Co., Ltd. Note 7: Organic-modified clay mineral: BENTONE 38 VCG, ex ELEMENTIS Note 8: Surface treated with KF-9909, ex Shin-Etsu Chemical Co., Ltd. | | | | | | | |

In the case where the present cosmetic composition is applied on the eyelashes, the purpose of the invention is to impart volume-boosting effect, long lash effect and gloss to the eyelashes. As seen in Table 3, the gloss of the cosmetic composition in Comparative Example 8 was insufficient. The volume of the cosmetic in Comparative Example 9 was insufficient and the long lash effect was poor. In contrast, the present cosmetic composition comprising the silicone-modified polysaccharide and the acryl-silicone graft copolymer showed the volume-boosting effect, the long lash effect and the gloss. In particular, when the ratio of the amount of the silicone-modified polysaccharide to the amount of the acryl-silicone graft copolymer was 3:1 to 1:3, the cosmetic obtained had all of the good volume-boosting effect, the good long lash effect and the good gloss. Particularly, as seen from a comparison between the cosmetic of Example 12 and the cosmetic of Comparative Examples 8 and 9, the volume was conspicuously improved by the combination of the silicone-modified polysaccharide and the acryl-silicone graft copolymer. This is a synergistic effect attained by the combination of the silicone-modified polysaccharide and the acryl-silicone graft copolymer.

### [Example 16]

### Mascara formulation (W/O)

### <Preparation of a mascara>

A: Components (1) to (12) were heated to 80 degrees C and mixed uniformly.
B: Components (13) to (15) were heated to 80 degrees C and mixed uniformly.
C: The mixture obtained in the step B was added to the mixture obtained in the step A, emulsified and, then, cooled to room temperature.
D: The mixture obtained in the step C was filled in a container to obtain a mascara.

**[Table 4]**

| | Component | Example 16 |
|---|---|---|
| 1. | Silicone-modified polysaccharide solution A ^{(Note 1)} | 7 |
| 2. | Acryl-silicone graft copolymer solution A ^{(Note 2)} | 7 |
| 3. | Branched polyether-modified silicone ^{(Note 6)} | 1.5 |
| 4. | Organic-modified clay mineral ^{(Note 7)} | 4 |
| 5. | Dextrinic acid ester ^{(Note 9)} | 2 |
| 6. | Ceresin | 5 |
| 7. | Microcrystalline wax | 5 |
| 8. | Propylene carbonate | 2 |
| 9. | Isododecane | Balance |
| 10. | Silicone composite powder ^{(Note 10)} | 3 |
| 11. | Silicone-treated ^{(Note 8)} black iron oxide | 5 |
| 12. | Silicone-treated ^{(Note 8)} talc | 5 |
| 13. | Purified water | 12 |
| 14. | 1,3-butylene glycol | 3 |
| 15. | Phenoxyethanol | 0.3 |
| | Total | 100 |

| | | |
|---|---|---|
| Note 9: Dextrinic acid ester: Leopal KL 2, ex Chiba Flour Milling Co., Ltd. Note 10: Silicone composite powder: KSP-105, ex Shin-Etsu Chemical Co., Ltd. | | |

The mascara had excellent adhesion, a volume-boosting effect, a long lash effect, gloss and a make-up retentivity.

### [Example 17]

### Mascara formulation (O/W)

### <Preparation of a mascara>

A: Components (1) to (5) were heated to 80 degrees C and mixed uniformly.
B: Components (6) to (13) were heated to 95 degrees C and mixed uniformly.
C: The mixture obtained in the step B was added to the mixture obtained in the step A, emulsified and, then, cooled to room temperature.
D: The mixture obtained in the step C was filled in a container to obtain a mascara.

**[Table 5]**

| | Component | Example 17 |
|---|---|---|
| 1. | Purified water | Balance |
| 2. | 1,3-Butylene glycol | 8 |
| 3. | Triethanolamine | 1 |
| 4. | Methylparaben | 0.2 |
| 5. | Acrylic acid polymer emulsion ^{(Note 11)} | 20 |
| 6. | Silicone-modified polysaccharide solution A ^{(Note 1)} | 0.6 |
| 7. | Acryl-silicone graft copolymer solution A ^{(Note 2)} | 0.4 |
| 8. | Polyoxyethylene sorbitan monooleate | 1.5 |
| 9. | Glyceryl stearate | 0.5 |
| 10. | Stearic acid | 2 |
| 11. | Beeswax | 7 |
| 12. | Carnauba Wax | 5 |
| 13. | Black iron oxide | 8 |
| | Total | 100 |

| | | |
|---|---|---|
| Note 11: Acrylic acid polymer emulsion: Yodosol GH 800 F, ex Akzo Nobel | | |

The mascara had an excellent adhesion, gloss and a make-up retentivity.

### [Example 18]

### Lipstick

### <Preparation of a lipstick>

A: Components (1) to (6) were heated to 90 degrees C and mixed uniformly.
B: Components (7) to (13) were added to the mixture obtained in the step A, heated to 70 degrees C and mixed uniformly.
C: Components (14) to (20) dispersed with three-roll mill were added to the mixture obtained in the step B, heated to 70 degrees C, mixed uniformly and, then, cooled to room temperature.
D: The mixture obtained in the step C was filled in a container to obtain a lipstick.

**[Table 6]**

| | Component | Example 18 |
|---|---|---|
| 1. | Candelilla wax | 8.6 |
| 2. | Polyethylene | 5 |
| 3. | Microcrystalline wax | 0.8 |
| 4. | Silicone wax ^{(Note 12)} | 8.2 |
| 5. | Macadamia nut oil | 6.6 |
| 6. | Isotridecyl isononanoate | 4.5 |
| 7 . | Silicone-modified polysaccharide solution B ^{(Note 13)} | 8. 3 |
| 8. | Arylic-silicone graft copolymer solution B ^{(Note 14)} | 33 |
| 9. | Branched polyglycerin-modified silicone ^{(Note 15)} | 0.5 |
| 10. | Decamethylcyclopentasiloxane | Balance |
| 11. | Methylparaben | 0.2 |
| 12. | Phenoxyethanol | 0.2 |
| 13. | Mica | 7.3 |
| 14. | Diisostearyl malate | 6 |
| 15. | Red No.201 | 0.3 |
| 16. | Red No.202 | 0.4 |
| 17. | Yellow No.4 | 1.2 |
| 18. | Silicone-treated ^{(Note 16)} titanium oxide | 2.9 |
| 19. | Silicone-treated ^{(Note 16)} black iron oxide | 0.2 |
| 20. | Silicone-treated ^{(Note 16)} red iron oxide | 0.7 |
| | Total | 100 |

| | | |
|---|---|---|
| Note 12: Silicone wax: KP-561P, ex Shin-Etsu Chemical Co., Ltd. Note 13: Silicone-modified polysaccharide solution B:TSPL-30-D5/a solid content 30 %, ex Shin-Etsu Chemical Co., Ltd. Note 14: Arylic-silicone graft copolymer solution B:KP-545/a solid content 30%, ex Shin-Etsu Chemical Co., Ltd. Note 15: Branched polyglycerin-modified silicone:KF-6104, ex Shin-Etsu Chemical Co., Ltd. Note 16: Silicone treatment: KP-574, ex Shin-Etsu Chemical Co., Ltd. | | |

The lipstick had an excellent adhesion, gloss and a make-up retentivity. Therefore, the present cosmetic composition can also be used as non-aqueous oily cosmetics such as a gloss, a stick concealer and a pouring foundation.

### [Example 19]

### Hair cream

### <Preparation of a hair cream>

A: Components (1) to (4) were mixed uniformly.
B: Components (5) to (9) were mixed uniformly.
C: The mixture obtained in the step B was added to the mixture obtained in the step A and emulsified, to which components (10) and (11) were then added and mixed uniformly.
D: The mixture obtained in the step C was filled in a container to obtain a hair cream.

**[Table 7]**

| | Component | Example 19 |
|---|---|---|
| 1. | Crosslinked polyether-modified silicone ^{(Note 17)} | 3 |
| 2. | Crosslinked dimethylpolysiloxane ^{(Note 18)} | 1 |
| 3. | Branched polyether-modified silicone ^{(Note 4)} | 0.2 |
| 4. | Dimethylpolysiloxane, 6CS | 8 |
| 5. | 1,3-Butylene glycol | 8 |
| 6. | Ethanol | 5 |
| 7. | Sodium citrate | 0.2 |
| 8. | Sodium chloride | 0.5 |
| 9. | Purified water | Balance |
| 10. | Silicone-modified polysaccharide solution B ^{(Note 13)} | 0.4 |
| 11. | Arylic-silicone graft copolymer solution B ^{(Note 14)} | 0.4 |
| | Total | 100 |

| | | |
|---|---|---|
| Note 17: Crosslinked polyether-modified silicone:KSG-210, ex Shin-Etsu Chemical Co., Ltd. Note 18: Crosslinked dimethylpolysiloxane: KSG-15, ex Shin-Etsu Chemical Co., Ltd. | | |

The hair cream gave gloss to the hair and had a good feeling in use.

### [Example 20]

### Hair wax

### <Preparation of a hair wax>

A: Components (11) to (17) were heated to 80 degrees C and mixed uniformly.
B: Components (1) to (10) were heated to 90 degrees C and mixed uniformly.
C: The mixture obtained in the step B was added to the mixture obtained in the step A, emulsified, cooled to room temperature and, then was neutralized by adding component (18).
D: The mixture obtained in the step C was filled in a container to obtain a hair wax.

**[Table 8]**

| | Component | Example 20 |
|---|---|---|
| 1. | Silicone-modified polysaccharide solution B ^{(Note 13)} | 2 |
| 2. | Arylic-silicone graft copolymer solution B ^{(Note 14)} | 3 |
| 3. | Decamethylcyclopentasiloxane | 10 |
| 4. | Candelilla wax | 14 |
| 5. | Microcrystalline wax | 6 |
| 6. | Isostearic acid POE glyceryl | 2 |
| 7. | Glycerin monostearate | 3 |
| 8. | PEG-11 methyl ether dimethicone ^{(Note 19)} | 2 |
| 9. | Stearic acid | 2 |
| 10. | Ethylhexyl methoxycinnamate | 0.1 |
| 11. | Propylene glycol | 6 |
| 12. | 1,3-Butylene glycol | 6 |
| 13. | Carboxyvinyl polymer | 0.3 |
| 14. | Methylparaben | 0.2 |
| 15. | Phenoxyethanol | 0.3 |
| 16. | Trisodium edetate | Proper quantity |
| 17. | Purified water | Balance |
| 18. | 10% aqueous solution of potassium hydroxide | 3.0 |
| | Total | 100 |

| | | |
|---|---|---|
| Note 19: PEG-11 methyl ether dimethicone: KF-6011P, ex Shin-Etsu Chemical Co., Ltd. | | |

This hair wax gave gloss to the hair and had a good hair-setting ability.

### INDUSTRIAL APPLICABILITY

The present cosmetic composition has very good adhesion. The cosmetics obtained from the present cosmetic composition have an excellent volume-boosting effect of the eyelashes and provide long lash effect and gloss to eyelashes without fibers. Therefore, the present cosmetic composition is particularly suitable as hair cosmetics, such as eyelash cosmetics. Further, the present cosmetic composition is suitable as non-aqueous oily cosmetics, such as a gloss, a stick concealer and a pouring foundation.

## Claims

1. A cosmetic composition comprising a silicone-modified polysaccharide and an acryl-silicone graft copolymer.

2. The cosmetic composition according to claim 1, wherein the amount of the silicone-modified polysaccharide is 0.1 to 25 mass % and the amount of the acryl-silicone graft copolymer is 0.1 to 31 mass %, based on the total mass of the cosmetic composition.

3. The cosmetic composition according to claim 1 or 2, wherein the polysaccharide in the silicone-modified polysaccharide is pullulan.

4. The cosmetic composition according to any one of claims 1 to 3, further comprising a nonionic surfactant.

5. The cosmetic composition according to any one of claims 1 to 4, further comprising a wax.

6. The cosmetic composition according to any one of claims 1 to 5, to be used for hair.

7. The cosmetic composition according to any one of claims 1 to 5, to be used for eyelashes.

8. The cosmetic composition according to any one of claims 3 to 7, wherein the silicone-modified polysaccharide has at least one silicone residue which is represented by the following formula (1) and which is bonded to a hydroxyl group on a glucose unit of pullulan, wherein the average number of the silicone residue bonded to one glucose unit is 0.5 to 2.5, 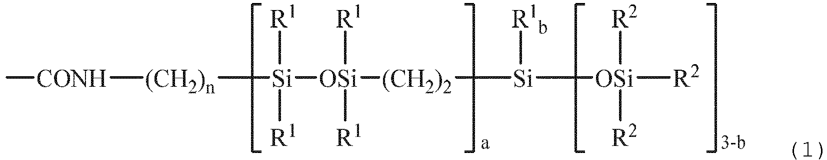 wherein R¹ is, independently of each other, an unsubstituted or substituted monovalent hydrocarbon group having 1 to 10 carbon atoms, R² is, independently of each other, an unsubstituted or substituted monovalent hydrocarbon group having 1 to 10 carbon atoms or a siloxy group represented by -OSi(R³)₃, R³ is, independently of each other, an unsubstituted or substituted monovalent hydrocarbon group having 1 to 8 carbon atoms, n is an integer of from 1 to 10, a is 0 or 1 and b is 0, 1 or 2, and the compound has an average molecular weight of 50,000 to 10,000,000.
